# EUROPEAN PATENT APPLICATION

(11) **EP 4 115 935 A2**
(43) Date of publication of application: **11.01.2023**
(21) Application number: 22178616.3
(22) Date of filing: 13.06.2022
(51) Int. Cl.: A61M 25/00

(54) **EXPANDABLE POLYMERIC FLARE TIP CATHETER AND METHODS OF FABRICATING SAME**

(30) Priority: 14.06.2021 US 202163210235 P
(71) Applicant: Neuravi Limited, Galway H91 K5YD (IE)
(72) Inventor: KEATING, Karl, Galway, H91 K5YD (IE); KELLY, Ronald, Galway, H91 K5YD (IE); CASEY, Brendan, Galway, H91 K5YD (IE); VALE, David, Galway, H91 K5YD (IE)
(74) Representative: Carpmaels & Ransford LLP

(57) **Abstract**

The designs herein can be for a clot retrieval catheter with a large bore lumen and a distal tip expandable to a diameter larger than that of the guide or sheath through which it is delivered. The designs can have a polymeric flared tip with atraumatic properties and the ability to flexibly expand when ingesting a clot. The tip can have a plurality of axial ribs giving stiffness to certain circumferential regions of the tip for facilitating repeatable collapse when retracted back into the guide or sheath. The tip can also have a metallic support frame within the flared tip for aiding in more gradual compression of clots during aspiration and retrieval with a stentriever. The catheter frame and tip can be sufficiently flexible to navigate highly tortuous areas of the anatomy and recover to maintain the inner diameter of the lumen when displaced in a vessel.

## Description

### Field of Invention

The present invention generally relates devices and methods for removing acute blockages from blood vessels during intravascular medical treatments. More specifically, the present invention relates to retrieval catheters with expandable tips into which an object or objects can be retrieved.

### Background

Clot retrieval catheters and devices are used in mechanical thrombectomy for endovascular intervention, often in cases where patients are suffering from conditions such as acute ischemic stroke (AIS), myocardial infarction (MI), and pulmonary embolism (PE). Accessing the neurovascular bed in particular is challenging with conventional technology, as the target vessels are small in diameter, remote relative to the site of insertion, and highly tortuous. Traditional devices are often either too large in profile, lack the deliverability and flexibility needed to navigate particularly tortuous vessels, or are not effective at removing a clot when delivered to the target site.

Many existing designs for aspiration retrieval catheters are often restricted to, for example, inner diameters of 6Fr or between approximately 0.068 - 0.074 inches. Larger sizes require an even larger guide or sheath to be used, which then necessitates a larger femoral access hole to close. Most physicians would prefer to use an 8F guide / 6F sheath combination, and few would be comfortable going beyond a 9F guide / 7F sheath combination. This means that once at the target site, a clot can often be larger in size than the inner diameter of the aspiration catheter and must otherwise be immediately compressed to enter the catheter mouth. This compression can lead to bunching up and subsequent shearing of the clot during retrieval. Firm, fibrin-rich clots can also become lodged in the fixed-mouth tip of these catheters making them more difficult to extract. This lodging can result in softer portions breaking away from firmer regions of the clot.

Small diameters and fixed tip sizes are also less efficient at directing the aspiration necessary to remove blood and thrombus material during the procedure. The suction must be strong enough such that any fragmentation that may occur as a result of aspiration or the use of a mechanical thrombectomy device cannot migrate and occlude distal vessels. When aspirating with a fixed-mouth catheter, a significant portion of the aspiration flow ends up coming from vessel fluid proximal to the tip of the catheter, where there is no clot. This significantly reduces aspiration efficiency, lowering the success rate of clot removal.

Large bore intermediate and aspiration catheters, and those with expandable distal tips, are therefore desirable because they can provide a large distal mouth to accept a clot with minimal resistance. The bore lumen of these catheters can be nearly as large as the guide and/or sheath through which they are delivered, and the expandable tip can expand to be larger still. When a clot is captured and drawn proximally into a tip with a funnel shape, the clot can be progressively compressed during retrieval so that it can be aspirated fully through the catheter and into a syringe or cannister. An expandable tip can be, for example, of polymeric construction to provide a softer, more clinically atraumatic vessel crossing profile with the ability to maintain an inner diameter and recover its shape when displaced laterally. However, designs with fixed frames or expanded sizes do not allow for additional expansion of the tip, which can complicate the interaction with and reception of a clot during ingestion.

Combining the clinical needs of these catheters without significant tradeoffs can be tricky, and many contemporary designs fall short in a number of categories. Catheter designs attempting to overcome the above-mentioned design challenges would need to have a large bore and an expandable tip with sufficient hoop strength in the expanded state to resist aspiration forces without collapse while having a structure capable of folding down consistently and repeatably when retrieved into an outer guide and/or sheath. The tip structure needs to have the flexibility and elasticity to survive the severe mechanical strains imparted when navigating the tortuous vasculature, while also being capable of expanding elastically as a clot is ingested for better interaction with and retention of the clot. Axially, the tip shape must maintain good pushability so that it can be advanced within an outer sheath, and in the expanded state in a vessel with minimal tendency to further over-expand outward when placed in compression. Such over-expansion can increase the delivery forces for the catheter and can also result in the aspiration catheter binding within the outer guide or sheath through which it is delivered. Further expansion in a vessel can damage vessel walls or snag when advanced.

The present designs are aimed at providing an improved retrieval catheter with an expansile flared tip capable of addressing the above-stated deficiencies.

### Summary

The designs herein can be for a clot retrieval catheter with a large bore lumen and a distal mouth that when advanced beyond the distal end of, can expand to a diameter larger than that of the guide or sheath through which it is delivered. The designs can have a polymeric flared tip for atraumatic properties and the ability to flexibly expand when ingesting a clot. The tip can have a plurality of axial ribs giving stiffness to certain circumferential regions of the tip for facilitating repeatable collapsing inserting into or retracting back through the guide or sheath. Some designs can also utilize a laser cut supporting frame within the flared tip for more gradual compression of clots during aspiration of during clot retrieval with a stentriever. The catheter frame and tip can be sufficiently flexible to navigate highly tortuous areas of the anatomy and recover to maintain the inner diameter of the lumen when displaced in a vessel.

The clot retrieval catheter may have a tubular large bore elongate body with an internal lumen and longitudinal axis extending therethrough. A large central catheter lumen can allow for enhanced aspiration efficiency and a low friction inner liner for the passage of guidewires, microcatheters, stent retrievers, and other such devices. The elongate body can have a metallic reinforcing layer that is braided, coiled, and/or laser cut to support one or more outer polymeric jackets. The tubular body can terminate at a distal end, at which an expandable polymeric flared tip can be integrally-formed or fixedly connected. The tip can be configured to expand from a collapsed delivery configuration to an expanded deployed configuration when extended beyond the distal end of an access sheath or intermediate catheter near or at the site of an occlusive thrombus.

In some examples, the catheter can have a 6F shaft and a flared tip having a maximum inner diameter a range between approximately 0.088 inches and approximately 0.098 inches in the expanded deployed configuration. The tip and catheter can then be used with a common 0.087 inch inner diameter guide sheath with the pushability to navigate through tortuous anatomy and sufficient tensile strength to withstand retraction through the same sheath having captured a clot with at least some stiff, fibrin rich components. The tip can also be flexible enough to further expand and cover the clot during retrieval.

Similarly, catheters with shafts in other common sizes, such as 5F or 7F, can also be envisioned with flared tip radial sizes which can scale accordingly. In other examples the flared tip can have a maximum inner diameter of approximately 0.110 inches or even 0.120 inches in the expanded deployed configuration, depending on the vessels being targeted. The tip can have a relatively short longitudinal length of approximately 25 mm for hinging around tortuous bends. In some cases, the tip can further have reinforcing metallic support arm struts or other frame reinforcements to enhance pushability and radial force.

At least a portion of the flared tip can have a wall thickness greater than a wall thickness of the elongate body to aid in compressing the clot and resisting the forces of aspiration. In some examples, a first wall thickness of at least a portion of the flared tip is different than a second wall thickness of a different portion of the flared tip. The ratio of an inner diameter of the elongate body to a maximum inner diameter of the flared tip when in the expanded deployed configuration can be in a range from approximately 0.55 - 0.90. In one example, the maximum inner diameter of the expanded flared tip can be 0.098 inches. In other examples, the maximum inner diameter can be up to 0.110 - 0.120 inches. The maximum inner diameter can be larger than the outer diameter of outer guide and/or sheath through which the catheter is delivered.

A polymeric distal bumper or lip can be added to the flared tip to make it more atraumatic and improve resilience when retracting stentrievers and other devices. In other examples, the tip can be made more atraumatic by having the distal end curve radially inward so the flared tip tapers to a final inner diameter less than a maximum inner diameter of the tip when in the expanded deployed configuration.

In some examples, the reinforcing layer of the elongate body can terminate axially at the proximal end of the flared tip, such that the tip itself is of strictly polymeric construction or has a reinforcing structure different from that of the elongate body. The supporting structure of the elongate body can be covered with a plurality of outer polymeric jackets. The flared tip and shaft outer jackets can be formed together or separately using injection molding, polymer reflow, or other suitable processes. In some examples, a compression molding process using a molding tool and die can be used. In other examples, the shaft and flared tip can be formed separately and attached to each other via adhesives or reflow. The outer polymer jackets can be made from suitable thermoplastic polymers of varying hardness to tailor the stiffness profile of the catheter shaft. In some examples, each jacket is formed from at least one of Esthane with 5% Carbosil, PU 80A, Pebax, Neusoft, Chronosil, Chronoprene, Chronoflex, Tecothane, or silicone.

The flared tip can have a plurality of axial ribs distributed around the circumference of the tip. The ribs can be localized columns of additional polymeric material configured to stiffen the tip along certain planes or in circumferential regions to facilitate predictable and consistent expansion and collapse of the tip between the expanded deployed configuration and the collapsed delivery configuration. The biased folding along the defined planes can give the tip the ability to maintain an inner diameter lumen for use with microcatheters, guidewires, and/or other auxiliary devices, or to collapse uniformly when advanced through an outer catheter luer.

The axial ribs of the flared tip can be internal and extend radially inward from the inner surface, or they can be external and extend radially outward from the outer surface. In another example, the ribs can extend through the wall thickness of the tip to protrude from both the interior and exterior surfaces. In a further example, some ribs can be internal and others external to create folding planes that can bias folding in some planes over others.

Other features can also be incorporated to aid the expansion and folding of the flared tip. In some examples, the tip can have one or more radial creases to influence the longitudinal locations where expansion and collapse occur. In other examples, the tip can have one or more radial creases which, similar to the ribs, can serve as hinge points to influence the circumferential locations where folding and wrap down occur.

In another example, a catheter for removing a clot from a blood vessel can have a substantially tubular elongate body having a proximal end, a distal end, and a lumen defining a longitudinal axis extending therethrough. The elongate body can have a reinforcing layer having a laser cut strut, coil, or braided structure, or a combination of these. The reinforcing layer can be encapsulated within one or more polymeric jackets as part of a reflow, dip coating, or plasma deposition process. The inner surface of the elongate body can have a low friction inner liner to aid in the passage of a guidewire, stentriever, or other auxiliary devices.

Connected to the distal end of the elongate body can be a flared tip forming a substantially conical or funnel shape when deployed and expanded. The flared tip can be of fully polymeric construction, or in some examples encapsulate an underlying metallic support frame. The support frame can have one or more support arms extending distally from the distal end of the elongate body. The arms can be independently sprung for increased flexibility or interconnected to improve the hoop strength of the tip. In some cases, the number of arms and their spacing around the flared tip can define bending planes to allow the distal end of the catheter to bend more freely for improved trackability.

In some examples, the flared tip can have a collapsed delivery configuration, and an expanded deployed configuration, and a plurality of axial ribs configured to add stiffness to facilitate expansion and folding of the flare tip between the expanded deployed configuration and the collapsed delivery configuration. The ribs can be in the interior or exterior of the polymer flared tip, or some combination of the two. The ribs can encase the support arms of the support frame to further bias folding of the tip for when it is wrapped down and retracted into an outer catheter or sheath.

A method for manufacturing the disclosed catheter designs can include the step of positioning an inner liner around a substantially tubular core mandrel. In one example, the core mandrel can be silver-plated copper and the liner can be a thin sleeve of PTFE or other low friction material with a strike layer for bonding. A longitudinal polymeric spine can be positioned around the inner liner to increase the tensile strength of the catheter shaft. A marker band having radiopaque properties can then be added to the distal end of the inner liner. Other marker bands can be positioned at various points along the catheter shaft.

Another step can involve a braided reinforcing layer being positioned around the core mandrel, inner liner, polymeric spine, and marker band. As with other examples, the braid can be multiple sections of varying composition and have features to transition the catheter body from having good column stiffness near the proximal end to excellent lateral flexibility near the distal end. A distal support frame can also be included with the reinforcing layer to give additional structure and support for the expandable tip. In some cases, the frame can be an extension from the reinforcing layer so there is no abrupt stiffness transition between the catheter body and the tip. In other examples, the frame can be formed separately and bonded with the reinforcing layer prior to forming the tip.

To bond the braided reinforcing layer, inner liner, polymeric spine, and marker band as a composite catheter shaft, a series of proximal polymeric jackets of varying stiffness can be reflowed over the structure and allow to flow through the interstitial spaces of the braid. Once the shaft is bonded, the core mandrel can be removed.

To form the expandable tip, a molding tool can be loaded proximally into the distal end of the pre-reflowed catheter shaft with the proximal jackets. The molding tool can have machined features to form ribs, creases, grooves, flanges, or other profiles into the jacket. A distal polymeric jacket and the profile of the flared tip can then be formed using the tool. The forming can be done using for example, an injection molding process or a compression molding process. Alternately, the tip could be formed in a separate molding process and attached to the shaft through reflow and/or adhesives. Once the expandable tip is formed in place, the molding tool can be removed from the catheter assembly

Other aspects and features of the present disclosure will become apparent upon reviewing the following detailed description in conjunction with the accompanying figures. Additional features or manufacturing steps can be included as would be appreciated and understood by a person of ordinary skill in the art.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is a view of a clot retrieval catheter having a flared tip with axial ribs according to aspects of the present invention;
FIGs. 2A-B show profiles of how the axial ribs can be used to control the expansion and collapsing of the flared tip according to aspects of the present invention;
FIGs. 3A-B illustrate cross sections of an example flared tip and catheter body according to aspects of the present invention;
FIG. 4 depicts an alternative configuration for a flared tip according to aspects of the present invention;
FIG. 5 shows another alternative configuration for a flared tip according to aspects of the present invention;
FIGs. 6A-D demonstrate examples of creases to facilitate flexibility and folding of flared tips according to aspects of the present invention;
FIGs. 7A-B illustrate another alternative catheter having a support frame for the flared tip according to aspects of the present invention;
FIGs. 8A-B depict a further example catheter having a support frame for the flared tip according to aspects of the present invention;
FIGs. 9A-9H illustrate method steps for the construction of a catheter with a polymer flared tip according to aspects of the present invention; and
FIG. 10 is a flow diagram for the construction of a catheter with a polymer flared tip according to aspects of the present invention.

### Detailed Description

Specific examples of the present invention are now described in detail with reference to the Figures, where identical reference numbers indicate elements which are functionally similar or identical. The figures illustrate a thrombectomy catheter with a large bore elongate shaft and an expansile polymeric distal tip. The shaft can have a reinforcing support structure overlaid with a plurality of polymeric jackets. One or more axial spines can be incorporated to improve the tensile strength and reduce the risk of shaft elongation for the catheter.

The expansile tip of the catheter can expand to a diameter larger than a guide or outer sheath used for delivery. The tip can be a substantially funnel shape when expanded and be flexible enough to further expand and better ingest and interact with a clot while retaining the tensile strength to withstand retraction into an outer catheter or guide sheath with firm clots. The tip can have features such as ribs and creases, which bias expansion and folding along certain planes in a predetermined and consistent manner so it can maintain an inner diameter for other devices while being capable of recovering its shape and expanding to an atraumatic vessel crossing profile when unconstrained. In some examples, the tip can also encapsulate a metallic support frame to stiffen certain sectors of the tip circumference.

Accessing the various vessels within the vascular, whether they are coronary, pulmonary, or cerebral, involves well-known procedural steps and the use of a number of conventional, commercially-available accessory products. These products, such as angiographic materials, mechanical thrombectomy devices, microcatheters, and guidewires are widely used in laboratory and medical procedures. When these products are employed in conjunction with the devices and methods of this invention in the description below, their function and exact constitution are not described in detail. While the description is in many cases in the context of thrombectomy treatments in intercranial arteries, the disclosure may be adapted for other procedures and in other body passageways as well.

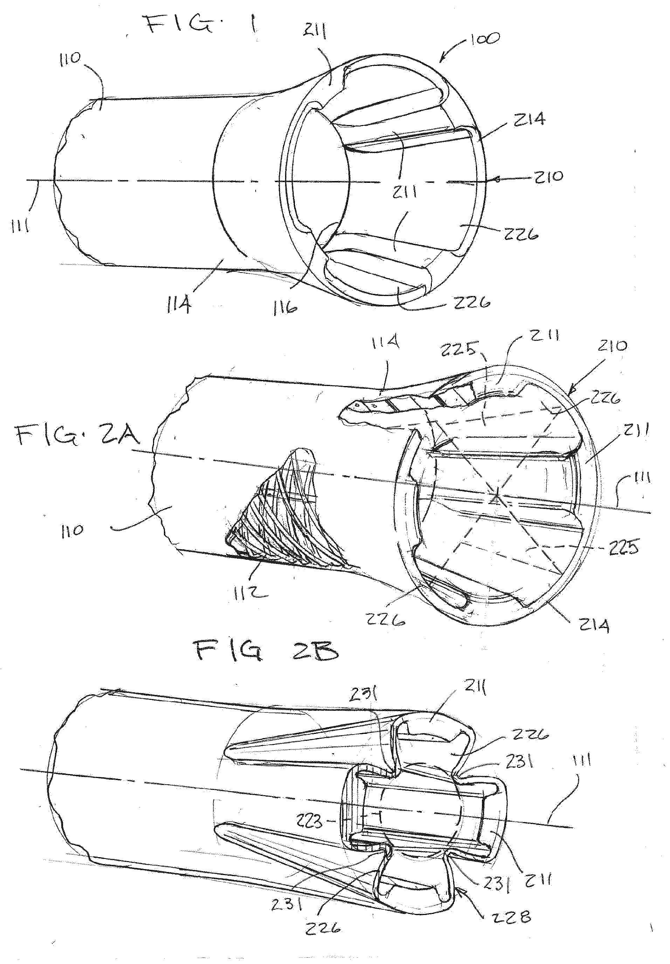

Turning to the figures, FIG. 1 illustrates the distal portion of a clot retrieval catheter 100 having an elongate body 110 proximal catheter shaft and a distal expansile flared tip 210. The catheter 100 can be navigated to a target site in the vascular using standard interventional techniques and commercially available ancillary devices such as an access catheter, balloon guide catheter, and/or guidewires.

The catheter shaft can be a substantially tubular elongate body 110 having a longitudinal axis 111 and defining a large inner limen 116. The catheter lumen 116 can be used for the delivery of auxiliary devices, such as microcatheters and stentrievers, and can also be used to direct aspiration distally through the expansile flared tip 210. The structure of the shaft body 110 can be, for example, a polymer and/or metal braid reinforcing support structure 112 with an internal low friction liner and outer polymer jackets that can be reflowed into the braid structure during manufacturing.

When used herein, the terms "tubular" and "tube" are to be construed broadly and are not limited to a structure that is a right cylinder or strictly circumferential in cross-section or of a uniform cross-section throughout its length. By way of example, the tubular elongate body shaft is generally illustrated as a substantially right cylindrical structure but can also assume a tapered or curved outer surface without departing from the scope of the present invention.

The flared tip 210 provides a wide mouth and a funnel profile for more gradual compression of a clot during retrieval, either through aspiration or with the aid of a stentriever or similar device. Gradual compression can lead to better management and retention of captured clots. The funnel profile of the expanded tip 210 can also at least partially impede proximal fluid from entering the tip during aspiration and retrieval of the clot, allowing for more efficient direction of the aspiration force to the clot face while preventing the distal migration of clot fragments of other debris during the procedure.

The example tip 210 shown in FIG. 1 has four internal axial ribs 211 spaced 90 degrees apart around the longitudinal axis 111 and extending from the distal end 114 of the elongate body 110 to the distal end 214 of the flared tip 210 to bias flexure during folding and expansion of the tip. In other examples, the ribs can extend only part of this distance. This design creates four thinner membrane sections 226 between the ribs 211. The thinner membrane sections 226 can give the tip excellent lateral flexibility and are reinforced by the ribs 211 to prevent collapse of the tip under aspiration.

This arrangement is shown by way of example, not limitation. It can be appreciated that the number of ribs and membrane sections can be varied to obtain other desired properties. An increase in the number of ribs can improve folding repeatability and resistance to collapse under aspiration. Fewer ribs can allow additional space for the collapsed profile of the tip to reside while increasing the circumference allotted to the membrane sections, improving the overall flexibility of the tip for expansion during clot retrieval and folding during retraction and collapse.

FIGs. 2A and 2B show of how thickened rib sections 211 of the flare tip 210 allow it to fold and wrap down in a predetermined way. An array of thick ribs 211 and thin membrane 226 polymer sections can promote the longitudinal folding of the flared tip to collapse into an outer catheter for delivery to or withdrawal from a target deployment area of a blood vessel. The thicker rib sections 211 enhance pushability of the tip by adding rigidity while the thinner membrane sections 226 allow for folding due to their flexibility.

Similar to FIG. 1, the example shown has four internal axial ribs 211 spaced 90 degrees apart around the longitudinal axis 111 and extending from the distal end 114 of the elongate body 110 to the distal end 214 of the flared tip 210. This configuration creates four membrane sections 226 interspersed between the ribs 211 and two folding planes 225 passing through the longitudinal axis 111 offset from each other by 90 degrees. When collapsed within, or retracted into, an outer catheter or sheath the tip can fold along these planes creating pleats 231 in the axial direction of the membrane sections 226 of the tip along a radially contracting folding profile 227.

Consistent collapsibility is important for durability and ease of use. For example, when first loading into an outer sheath for delivery, the catheter can be advanced through a profiled introducer tool that will uniformly collapse the tip 210 to fit inside an outer sheath such as a balloon guide catheter. The inner diameter 223 of the collapsed tip is preserved so the inner lumen is maintained and smaller introducers or microcraters and guidewires can be advanced through it, as seen in FIG. 2B. The collapsed profile 228 can thus form a cross or star profile with diametrically opposed peaks without crushing or blocking the lumen, and the user will not need to be concerned with the independent usage of tools during a procedure.

These shapes can be advantageous when using a telescoping approach to reach a target vessel location. Telescoping allows the user to first advance a smaller more trackable catheter to the treatment location and then advance a larger catheter over it, using the smaller catheter/guidewire as a rail to guide advancement of the larger catheter.

Alternatively, a loading tool can be supplied on the shaft of the catheter so that it is advanced distally over the funnel of the flared tip to collapse the tip before inserting through the luer of an outer guide sheath. In another similar example, a split tool can be used to collapse the funnel through stretching. The tool halves can be joined together using thread locks, snap locks or with magnetic fastening features. This allows the user to attach, remove and reattach the tool as needed, for example, if the physician needs to do a second pass with the same catheter, the tool can be reattached to allow collapse of the flared tip for a second advancement through an outer guide after a first pass has been completed.

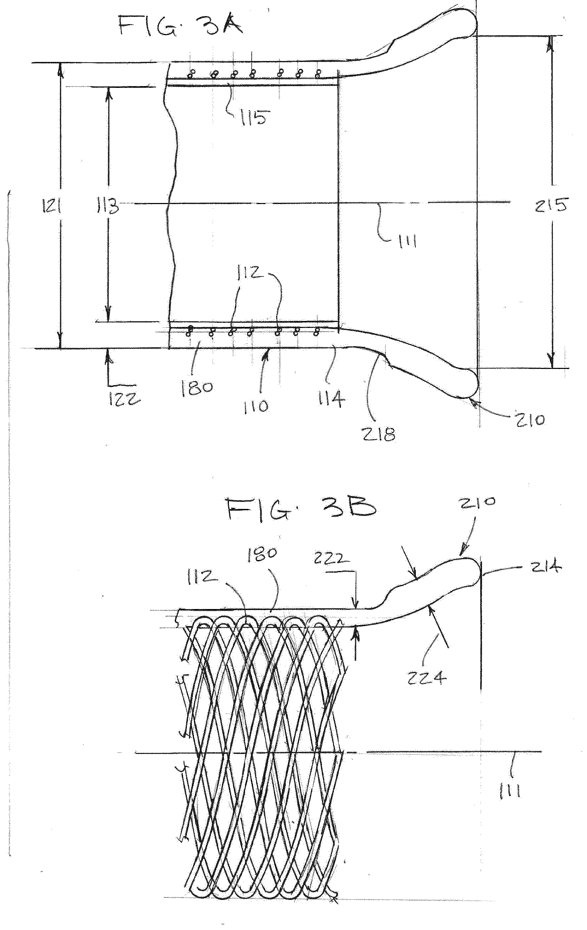

As shown in the cross section in FIG. 3A, the elongate body 110 forming the catheter shaft can have a composite construction with an inner liner 115, a reinforcing layer 112 disposed around the liner, and an outer polymer jacket 180. The catheter can be assembled on a flared molding tool or mandrel during manufacturing so that the jacket 180 can be injection molded, compression molded, and/or reflowed to form the flared tip 210 extending distal to the distal end 114 of the elongate body.

The catheter can have a large bore through the elongate body 110 of the shaft while remaining compatible with typical sheaths used for neurovascular thrombectomy procedures. Improvement of the disclosed designs over traditional fixed bore/mouth catheters is the combination of the large internal bore with a distal flared tip 210 capable of expanding to a greater diameter than the outer catheter or guide sheath through which it is deployed. The soft, elastic flared tip 210 is also very atraumatic and can easily deform and collapse to enter the outer catheter or guide sheath for delivery and retrieval. The collapse can further be aided by incorporating one or more radial creases 218 into the tip 210 profile. A radial crease 218 can be formed through molding, heat forming, or by using a second heat shrink ring that compresses at a faster rate than an outer heat shrink sheath used for the tip. A crease 218 as shown just beyond the distal end 114 of the elongate body 110 can function as a pivot for the radial expansion of the flared tip.

To be compatible with many of the most widely adopted guides and/or sheaths, the inner diameter 113 of the catheter elongate body 110 and the inner diameter 215 of the flared tip 210 can be sized appropriately. For example, a 5F catheter targeting vessels approximately 2.0 mm in diameter can have a shaft inner diameter 113 of approximately 0.054 inches and an expanded tip 210 inner diameter 215 in a range from approximately 0.068 - 0.090 inches. Similarly, a 6F catheter targeting vessels approximately 2.3 - 3.4 mm in diameter can have a shaft inner diameter 113 of approximately 0.068 - 0.074 inches and an expanded tip 210 inner diameter 215 in a range from approximately 0.090 - 0.120 inches. A larger 8F catheter for less remote clots can have a shaft inner diameter 113 of approximately 0.082 - 0.095 inches and an expanded tip 210 inner diameter 215 in a range from approximately 0.090 - 0.188 inches. The upper limit of expanded tip diameters is limited by delivery forces when traversing within an outer guide or sheath. These common sizes can result in the ratio of the inner diameter 113 of the elongate body 110 to the maximum expanded inner diameter 215 of the flared tip 210 being in a range from approximately 0.55 - 0.90.

In one example, the inner diameter 113 of the elongate body for a 6F catheter of the designs presented herein can be approximately 0.071 inches. In the expanded deployed configuration, the flared tip 210 can have a maximum inner diameter 215 of approximately 0.098 inches. In some examples, the maximum inner diameter 215 can be as small as 0.088 inches or as large as 0.120 inches. In either case, the expanded tip can have a diameter greater than that of a standard 0.087 inch ID guide sheath often used for the delivery of this sized aspiration catheter. This allows the elongate body 110 of the catheter shaft to have a large bore while yielding even greater aspiration efficiency through the expanded tip.

A cross section view of a braided reinforcing layer 112 and tip profile of the outer polymer jacket 180 can be seen in FIG. 3B. The braid of the reinforcing layer 112 can have varied pitch, angles, and/or axial spacings to tailor the stiffness properties for different axial lengths of the catheter shaft. Certain angles can also allow additional radial expansion for ingesting stiff clots. The reinforcing layer 112 can also have multiple braided segments having different properties and/or materials. The reinforcing layer 112 can terminate at the distal end 114 of the elongate body 110, along with the inner liner 115.

The outer jacket 180 of the tip section can be extremely flexible for ease of expansion and collapse while having a soft and gently curved atraumatic distal end 214. The jacket materials can be, for example, low durometer urethanes which can flex easily against vessel walls and are durable and easy to manufacture. As with other examples, the dimensions and material properties of the outer jacket 180 can still be tailored for desired tip 210 qualities. For example, thicker tips may be combined with softer materials of 40 Shore A or lower, or thinner tips may be formed from stiffer materials of up to 55 Shore D or more. In some examples, a material such as Pebax with a hardness of approximately 35 Shore D can be used. In other examples where a slightly stiffer tip can offer more radial force, a Pebax with a hardness of approximately 55D can be substituted.

Other designs can feature an outer jacket 180 of the flared tip 210 formed from a combination of two materials. One material can have greater stiffness than the other to provide radial support. The softer material can collapse more easily for moving from an expanded configuration for aspiration to a collapsed configuration for delivery through the guide sheath,

Increasing the wall thickness 224 at or near the distal end 214 of the flared tip 210 can fortify and give additional structure to the mouth of the funnel shape so that the tip 210 can maintain its inner diameter 215 when navigating tortuous bends in the vasculature. A thicker distal portion can also improve the resilience of the tip when retracting stentrievers or other devices during a procedure.

In contrast, a thinner distal end 214 of the tip can function as a flanged profile to provide more atraumatic vessel crossing and prevent the tip from snagging on bifurcations and other vessel take-offs. A flange portion could also partially cover a clot during retraction through an outer catheter or guide sheath to further prevent it from becoming dislodged.

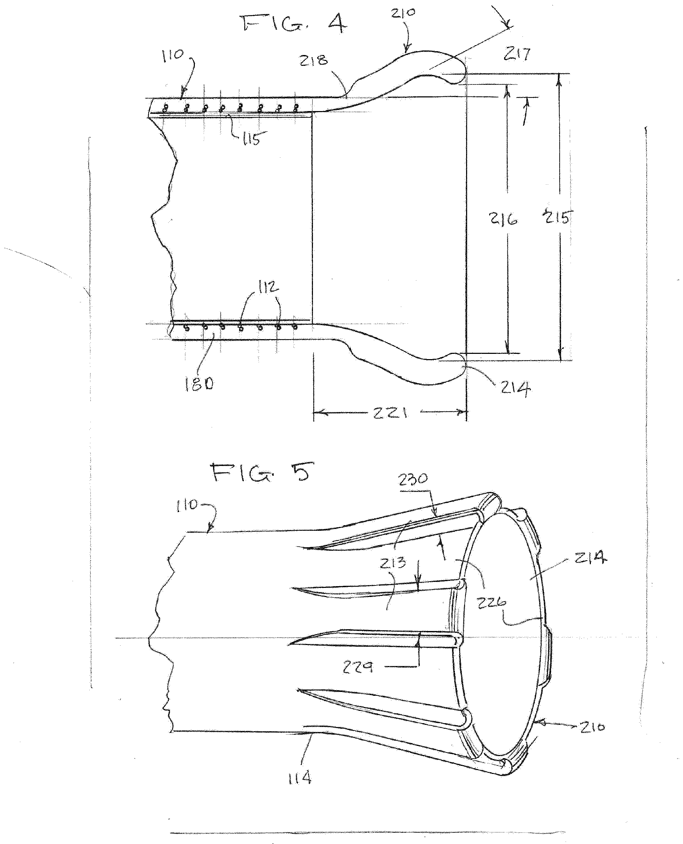

FIG. 4 illustrates another cross section of an example flared tip 210 in accordance with the design aspects disclosed herein. The tip can have a stepped change in wall thickness from that of the elongate body 110. In some examples, the wall thickness 122 of the elongate body can be in a range of approximately 0.0045 - 0.0060 inches. This thickness can increase to 0.0080 inches or more in the flared tip 210 distal of the termination of the reinforcing layer 112. Keeping the axial length 221 of the flared tip relatively short (approximately 20 - 30 mm) can allow the tip to hinge about the elongate body when being displaced in a vessel. A radial crease 218 can be added to aid in folding to a collapsed profile with less impact on the braid wires of the reinforcing layer 112.

The flared tip 210 can also have a distal end 214 which can curve radially inward for an atraumatic vessel crossing profile. As a result, the final inner diameter 216 at the distal end 214 of the tip 210 can be less than the maximum inner diameter 215 at some intermediate axial position. For example, a 6F catheter with a flared tip 210 having a maximum diameter of 0.095 inches similar to previously disclosed examples can taper to 0.090 inches at the distal end 214. This profile can help to prevent the flared tip from snagging on bifurcations and when the catheter is being advanced distally with the tip in the expanded configuration. A radially inward curve can also give additional structure and strength to the mouth of the tip for stentrievers or other devices when they are retracted back into the tip.

A shallow flare angle 217 of the funnel shape can lead to a more gradual taper of the tip 210 and increase the maximum inner diameter 215 for smooth interaction with a clot. This interaction can reduce both the axial and radial forces exerted on a clot, as it is more gradually ingested than it would be with steeper flare angles. The progressive and measured compression can also reduce the risk of bunching up and subsequent shearing of portions of the clot during retrieval.

Another example of an expansile flare tip having a variation of ribs is illustrated in FIG. 5. The flare tip illustrated can have six external axial ribs 213 protruding radially from the outer circumference of the funnel shaped tip 210 and framing six thinner membrane sections 226 between them. The ribs 213 can gently taper outward approximate the distal end 114 of the elongate body 110 and extend with the funnel profile to the distal end 214 of the flared tip. It can be appreciated that longitudinally shorter or longer ribs can be envisioned, as well as an increase or decrease in the number of ribs as previously mentioned.

Although the overall objective of adding axial ribs to the flared tip remains the same, a number of distinctions can be drawn between the influence of the internal ribs 211 of FIG. 1 and the external ribs 213 of FIG. 5. For example, internal ribs 211 can keep the exterior of the funnel profile of the tip 210 smooth for atraumatic vessel crossing and reduce the potential for snagging. However, the smooth surface can lead to a greater contact area with an outer catheter or sheath, which can increase friction during advancement or retraction.

In contrast, utilizing external ribs 213 can aid in the folding of the flared tip 210 during advancement through an outer catheter luer and reduce friction during advancement to a deployment location both within the outer catheter and within a target blood vessel. A flatter interior surface of the tip can also reduce the likelihood of a stentriever or other devices snagging on the ribs during retraction and provides a smooth interface for a clot during aspiration and ingestion.

Manufacturing challenges can also play a role in design choices between external 213 and internal ribs 211. As injection molding can be challenging with thin walled membrane sections 226, external ribs 213 can utilize an over molding process for a preformed tip, or compression molding to produce an integral tip. Depending on the quality of the initial compression molded tip, additional polymer reflow sub steps may also be required. On the other hand, internal ribs 211 can be formed through injection or compression molding, or through a controlled reflow process using an outer heat shrink sleeve and an inner profiled mandrel.

Variations in rib 213 width 229 and thickness 230 can also influence other properties of the flared tip 210. Wider and/or thicker ribs can lend more pushability and a higher resistance to radial collapse during aspiration. They can also improve tensile properties of the tip when the tip is expanded or interacting with a clot. Alternately, ribs 213 with less width can result in wider and thinner membrane sections between the ribs which can improve conformability for folding and a greater radial expansion capacity during clot retrieval. Greater expansion capabilities can reduce the forces necessary to capture and retrieve a clot, meaning managing clot ingestion and retention is improved over otherwise stiffer tips or frames.

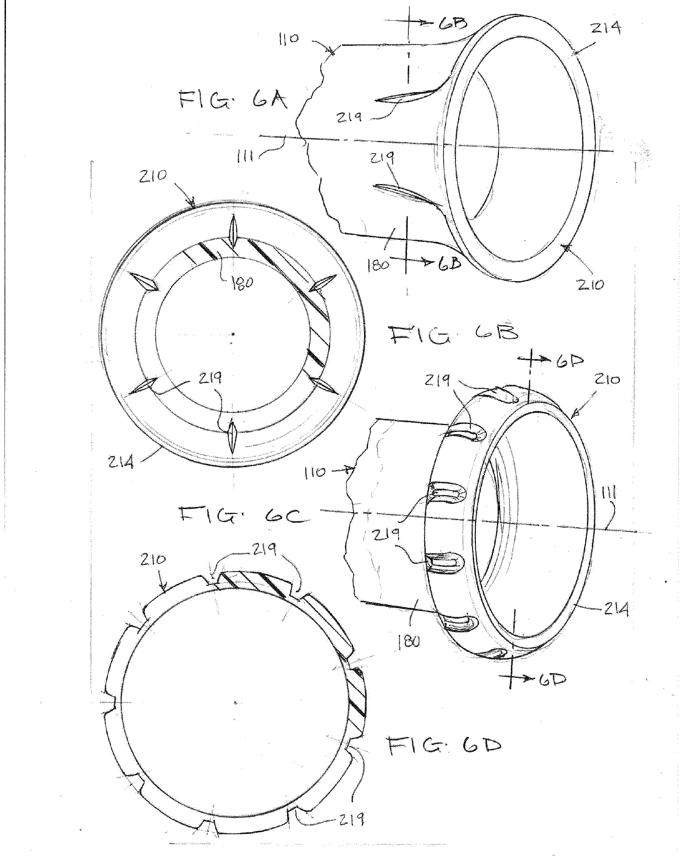

Other variations of the catheter flared tip 210 can be seen in FIG. 6A and 6C, along with the cross sections in FIG. 6B and FIG. 6D, respectively. Similar to the functioning of the ribs in other examples, longitudinal creases 219 can be formed into the flared tip 210 to promote the folding of the polymer to collapse into an outer catheter for delivery to the target deployment area of a blood vessel. The increased flexibility from the creases 219 can also reduce the stresses imposed on the wires of the reinforcing layer 112. A number of longitudinal creases 219 can be included with or without an array of radial creases 218. Spiral, curved, and/or other profiles of creases can also be incorporated into the design.

FIG. 6A and FIG. 6C. also show how the distal end 214 of the flared tip 210 can have a large, rounded shape for atraumatic vessel contact during advancement to a target site. This profile can also prevent snagging on bifurcating vessels such as the ophthalmic. A distal bumper or flange of additional material can also be included at the distal end 214 of the tip to aid in maintaining the inner diameter of the tip when navigating tortuous bends in the vasculature. A thick bumper can give additional structure to the tip for improved resilience when retracting stentrievers or other devices back into the tip.

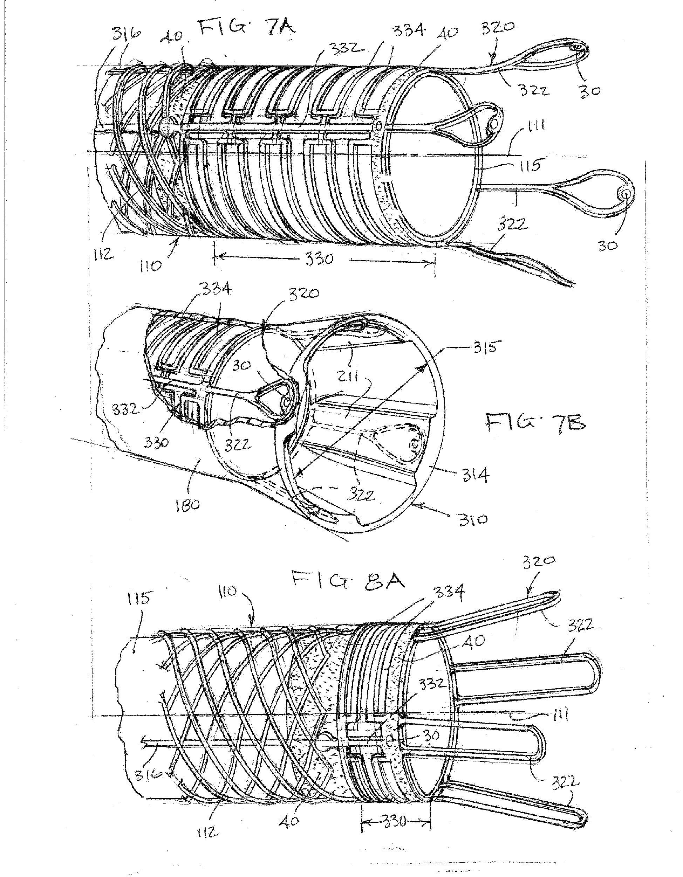

In the example illustrated in FIGs.7A-B and FIGs. 8A-B, the flared tip 310 can have an underlying support frame 320 reinforcing and giving further strength to the polymer of the tip. The support frame 320 can have one or more support arms 322 extending distal to the elongate body 110. The design in FIGs. 7A-B has four arms 322 equally spaced around the longitudinal axis 111 and terminating in distal loops or hoops. It can be appreciated that fewer or more support arms 322 can be used with different connection combinations with elongate body 110. When bending, or when the tip 310 is placed under compressive loads during retrieval of a clot, fewer rigid connections can give the tip added flexibility and the ability to deflect locally for a tighter grip on the captured clot. The hoops can incorporate radiopaque markers 30 to aid in visualization of the device during a procedure.

The support arms 322 can be laser cut integrally with the underlying reinforcing layer 112 of the elongate body 110, machined as separate members, or a combination of integral and independent members. FIG. 7B shows how the arms 322 can be encapsulated within internal axial ribs 211 of the flared tip 310.

The arms 322 can extend and flex independently as shown, or adjacent arms can circumferentially overlap or be otherwise connected. The arms do not need to be fixedly coupled if they overlap, meaning they can be interlaced and can slide and fold relative to each other as the tip 310 expands or contracts. Designs having more than four support arms 322 can be appreciated where additional arms sacrifice some tip flexibility while providing additional radial force and support to prevent the collapse of the tip under aspiration. Similarly, fewer arms 322 can be utilized in situations where a polymer of greater stiffness or thickness is utilized, or more substantial axial ribs 211 require less support. Fewer arms 322 can also bend more freely for improved trackability. In one example, the support frame 320 can have two support arms 322 laser cut and spaced 180 degrees apart.

In some examples, the elongate body 110 of the catheter shaft can transition from a braided reinforcing layer 112 to a distal hinged structure 330. The hinged structure 330 can be configured to bias bending of the flared tip 310 and distalmost portion of the elongate body to allow the tip to deflect away easily and effectively from vessel walls. As the large bore catheter and expanded tip are designed to be independently advanced distally in vessels, this hinging capability is important in particularly tortuous vessels, or when navigating several bends in quick succession. The hinged structure 330 can account for significant amounts of lateral displacement when in impinging on the vessel wall so the flared tip 310 does not excessively deform and close down or collapse into an irregular shape. This allows the tip to maintain its inner diameter and rapidly recover its expanded shape.

The hinged structure 330 can be laser cut hypotube of superelastic Nitinol or other suitable material with a series of circumferential ribs 334 connected to one or more axial spines 332. In one example, the structure can have two parallel spines 332 spaced 180 degrees apart which can facilitate deflection of the flared tip 310 along a plane passing through the two spines and the longitudinal axis 111. The spines can also be aligned with the axial spine 316 or spines of the elongate body for added resistance to tensile elongation. This structure can have a greater ability to flex than the more proximal braided reinforcing layer 112.

One or more radiopaque marker bands 40 can be added to identify important points along the device during use. In some examples, a marker band can serve as a joint to which the underlying reinforcing layer 112 and/or hinged structure 330 can be welded or otherwise adhered to. In other examples, the marker bands 40 can be formed integrally with the hinged structure 330 and used as attachment points for the shaft.

The support frame 320 of the flared tip 310 can be formed integrally with the hinge structure 330. This arrangement can allow for a smoother stiffness transition as at least some of the support arms 322 can extend distally from the hinge spines 322 as seen in FIG. 7A. A radiopaque marker band 40 can be positioned on the elongate body 110 and during assembly the laser cut support hinged structure 330 and tip support frame 320 can be threaded over the inner liner 115 and welded or otherwise bonded to the maker band with adhesives.

Similar to many current technologies, the reinforcing layer 112 of the catheter shaft can have a braided wire support structure as a primary backbone. The braided reinforcing layer 112 can be covered by an axial series of plastic tubular jackets 180, 182. The jackets can be made of various medical grade polymers, such as PTFE, poly ether block amide (Pebax^{®}), or Nylon. Materials can be chosen, for example, so that progressively more proximal segments are generally harder and less flexible (by durometer hardness, flexure modulus, etc.) as the proximal end of the catheter is approached. The tubular jackets 180, 182 in FIG. 7B can be reflowed over the reinforcing layer 112 and hinged structure 330, and the flared tip can be applied in a separate reflow or through a molding process. In many cases, the flared tip 310 can be an extension of the distalmost jacket 180.

An alternative hinged structure 330 and support frame 320 for the flared tip are illustrated in FIGs. 8A-B. Similar to other examples, marker bands 40 can serve as a joints to which the reinforcing layer 112 and hinged structure 330 can be attached. The support frame 320 can have a plurality of support arms 322 shaped as distal hoops. The support arms 322 can each have their own connection with the hinged structure 330 as shown so they can flex independently and more freely as they experience loads during a clot retrieval procedure. The flared tip 310 can have external axial ribs 213 sized so they can fully encapsulate the support arms and maintain the atraumatic profile of the tip. As in previous examples, the number of support arms can vary, and they can be evenly spaced around the longitudinal axis 111 so the funnel shape of the flared tip is rounded as opposed to potentially being stretched into an ovular profile. A rounded opening is preferred to better receive and gradually compress a clot during removal after it has been dislodged.

It should be noted that any of the herein disclosed catheter and flared tip designs can be used with one or more stentrievers. The combined stentriever retraction and efficient aspiration through the enlarged tip section in the expanded deployed configuration can act together to increase the likelihood of first pass success in removing a clot. The catheter can also direct the aspiration vacuum to the clot face while the stentriever will hold a composite clot (comprised of friable regions and fibrin rich regions) together preventing embolization and aid in dislodging the clot from the vessel wall. The funnel-like shape of the tip section can also reduce clot shearing upon entry to the catheter and arrest flow to protect distal vessels from the risk of new territory embolization.

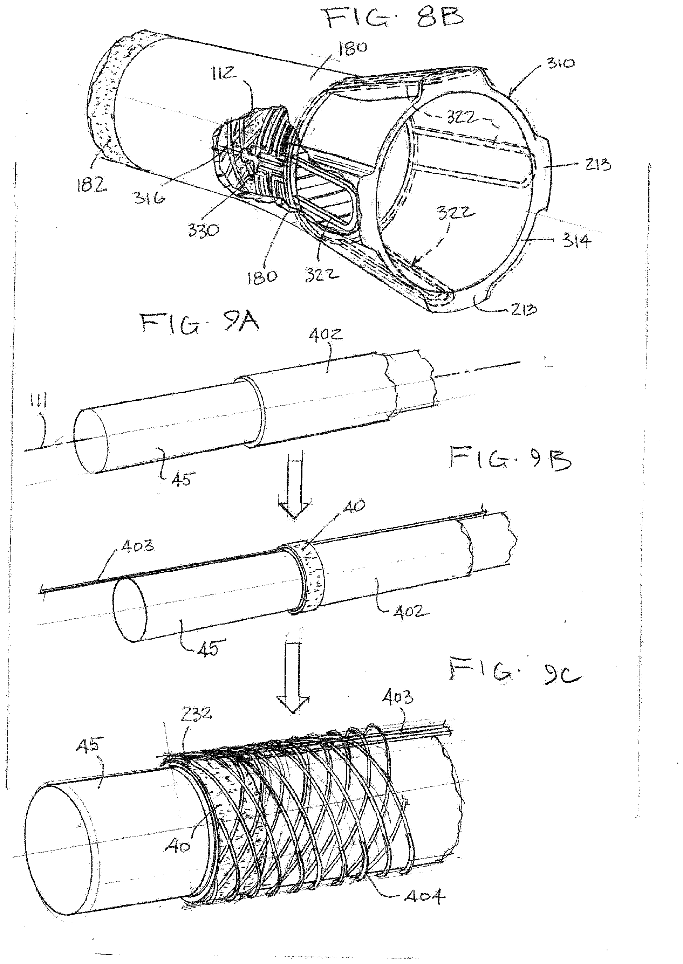

FIGs. 9A-H pictorially represent a method for the manufacture of a catheter having a substantially tubular composite shaft and an expandable polymeric flared tip at the distal end. FIG. 9A shows an inner liner 402 with a thin strike layer (not shown) to boost adhesion positioned around a cylindrical core mandrel 45. A strike layer can help reduce the risk of the liner 402 delaminating from the catheter assembly. For a nominal 6F size catheter shaft, the core mandrel 45 can have an outer diameter of approximately 0.071 inches, and the inner liner can be approximately 0.002 inches in thickness. The mandrel can often be silver-plated copper (SPC) as is commonly used for these applications. Alternatively, especially ductile materials (such as PEEK) can be used which stretch to neck down in diameter so that the mandrel can be removed after completion of the catheter assembly. Further mandrel materials can include nylon coated copper or nylon coated steel.

Referring to FIG. 9B, one or more longitudinal spines 403 can be positioned around and/or adhered to the liner 402. The one or more spines 403 can enhance pushability and tensile strength of the catheter shaft, and materials such as stainless steel, Nitinol, liquid crystal polymer (LCP), or Kevlar fibers can be used. A liquid crystal polymer can offer the highest tensile strength, while a stainless steel spine can offer the best pushability at the cost of some lateral flexibility. The spine 403 can run parallel to the longitudinal axis 111 as shown or can be a spiral or some other configuration.

The spine or spines 403 can be added prior to or after adding a marker band 40 near or at the distal end 414 of the assembly, as illustrated in FIG. 9B. Other marker bands can be positioned on other sections of the shaft for use as positional references during a procedure. The bands can be crimped, adhered, or otherwise bonded in place. The marker band 40 can be platinum or other suitable radiopaque substance, or alternatively be coated with a film visible under fluoroscopy. The reinforcing layer 404 for the shaft, which can be a wire braid, cut hypotube, or other construction can be applied over the core mandrel 45, inner liner 102, spine 403, and marker band 40 as in FIG. 9C. Alternatively, the reinforcing layer can be positioned over the liner but beneath the marker band and spine. A braided section, for example, can have different sections of stainless steel and/or Nitinol wire of varied thickness. The sections can also have differing braid densities, such as a mixed PPI in a range of 120 - 180.

As an option, some or all of the LCP spine can be replaced with a stainless steel and/or Nitinol spine, and the reinforcing layer 404 and metallic spine 403 can be welded to the marker band 40 to fix their respective locations. Additionally, the marker band 40 can be applied after braiding for the spine 403 to lie under. If a support frame 320 or hinged structure 330 for the flared tip are to be used (like that shown and described for FIGs. 7 and 8), they can also be slid over the underlying assembly and welded to the marker band 40 distal of the reinforcing layer 404. Alternately, this hinged structure can feature a braid welded to the marker band that has a different pattern than that used on more proximal portions of the shaft.

As also seen in FIG. 9C, a distal portion of the spine 403 can be looped through a cell wrapped around the distal end of the braided reinforcing layer 404 to form a loop 232 proximally give the shaft additional tensile strength. In other examples, the axial spine loop 232 can be fed through a more proximal opening in the braid. The spine 403 can thus have one length running on top and one length extending beneath of the reinforcing layer 404, or, can be interwoven through the braid openings in an undulating fashion. In many cases, once the loop 232 has been formed, what was the distal portion of the spine can extend proximally all the way to the luer at the proximal end of the catheter. In other examples, the end can be welded or adhered to one of the marker bands at an intermediate position on the shaft.

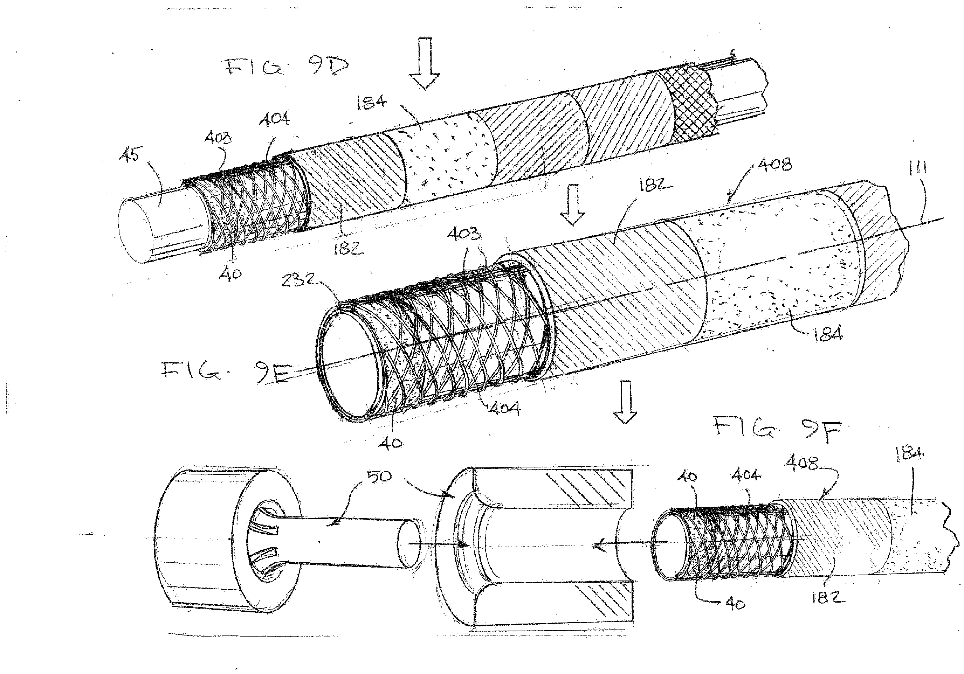

The method can continue in FIG. 9D with a series of proximal outer jackets 182, 184 of varying stiffness reflowed onto the shaft. The jackets can be allowed to flow through the interstitial spaces of the reinforcing layer 404 to bond the layers of the construct together. The series of polymer jackets can be butted together to form a continuous and smooth outer surface for the catheter shaft. The jackets 182, 184 can have differing durometer hardness and/or wall thicknesses. For example, a stiffer more proximal portion of the shaft can have a jacket with a thickness of approximately 0.004 inches and a hardness of around 72D. By contrast, a distal section requiring greater flexibility but where the underlying support braid is less dense can have a jacket with a wall thickness in the region of 0.006 inches and a hardness of around 35D.

FIG. 9E shows the core mandrel 45 removed after the reflow of proximal jackets 182, 184 to the shaft construct. As shown, a distal portion of the inner liner 402 and reinforcing layer 404 can remain uncovered (for a distance of approximately 5-10 mm) to be coated with a more distal jacket material as part of the flared tip forming operations.

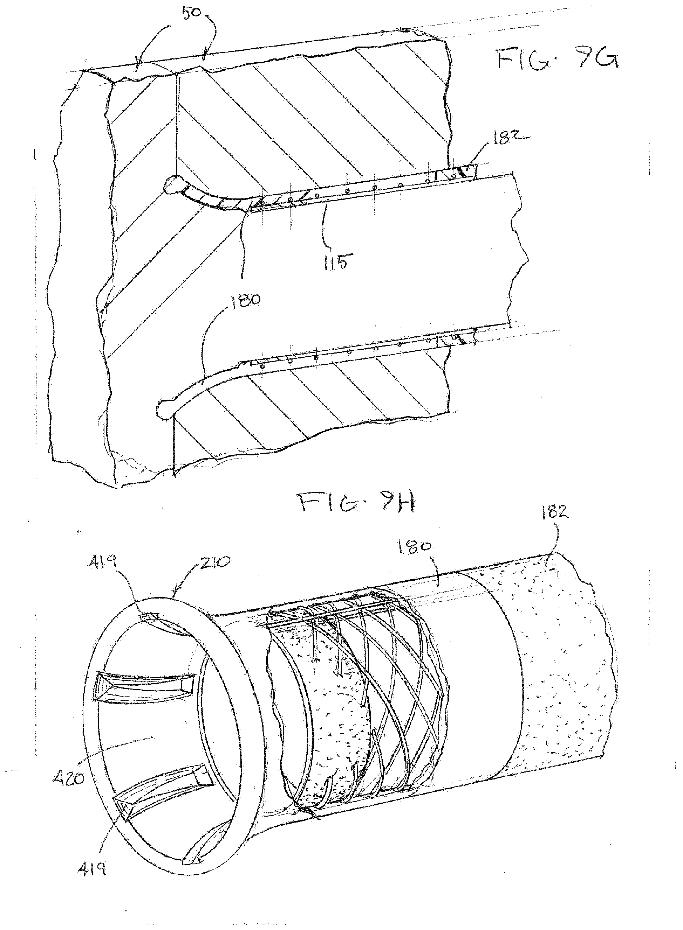

FIG. 9F then illustrates the injection molding tool 50 assembled with the catheter tip. The molding tool 50 can be flared and profiled machined features to give the flared tip axial ribs, creases, and other geometries. The distal jacket 180 material can then be overmolded onto the shaft 408. The jacket 180 can be mixed with additives, such as colorants, forming agents, or often radiopaque compounds. In one example, a thermoplastic with a hardness of approximately 35D - 55D can be overmolded with a radiopaque filler, such as titanium dioxide. The proximal end of the flared tip can be tapered down to the same wall thickness as the jackets of the shaft in the mold, thereby reducing the risk of warping or excessive flash (FIG. 9G).

It can be appreciated that the molding tool 50 illustrated for these method steps is just one of many possible profiles which can be used to form the flared tip 210. In other examples, the machined features of the mandrel can allow the reflowed or molded tip to have wide or very narrow membrane segments, a flanged edge at the distal end, or a thickened bumper section or lip at the distal end. These features can also be added using additional molding or reflow steps.

As an alternative to injection molding, a reflow tool (not shown) can be back loaded into the distal end of the pre-reflowed shaft assembly of FIG. 9E. The distal jacket 180 can then be threaded over the tool and the distal section of the braided assembly and reflowed. With the tool removed, the distal jacket 180 can be trimmed to length and inverted over itself multiple times to increase the effective wall thickness. The inverted jacket can then be loaded into a tool and profiled die and compression molded to the desired shape.

In another example, a flared mandrel (not shown) can be inserted into the distal end of the pre-reflowed shaft assembly of FIG. 9E. An extrusion of the distal jacket 180 can be threaded over the mandrel and the distal section of the braided assembly and reflowed. A sleeve of PTFE or other suitable low friction material can be applied to the shaft and used to control and arrest the proximal flow of the polymer of the distal jacket when it is reflowed. A heat shrink sleeve can be positioned around the flared mandrel and the section of the shaft to be reflowed with the distal jacket 180. The heat shrink sleeve can be FEP or another suitable copolymer and is used to help conform the material to the mandrel shape. If necessary, any excess material can be trimmed away to ensure the desired profile is attained.

Another method can involve using injection molding to form and finish the flared tip 210 separately from the shaft. This can have the advantage of insulating the completed shaft from the hazards of further heat and processing. The completed tip can then be attached to the shaft through adhesives or reflow.

Once the distal jacket 180 and flared tip 210 have been formed, the molding tool (or mandrel) can be removed from the assembly, as depicted in FIG. 9H. As with other examples, the tip 210 formed by the molding process can have creases 419, ribs, or other features for repeatable folding and increased tip flexibility. The interior of the flared tip can be coated with a hydrophilic material 420 to reduce surface friction and enhance the lubricity of the surface. A hydrophilic coating can further reduce the retraction forces when using auxiliary devices with the tip, or when a large clot is ingested.

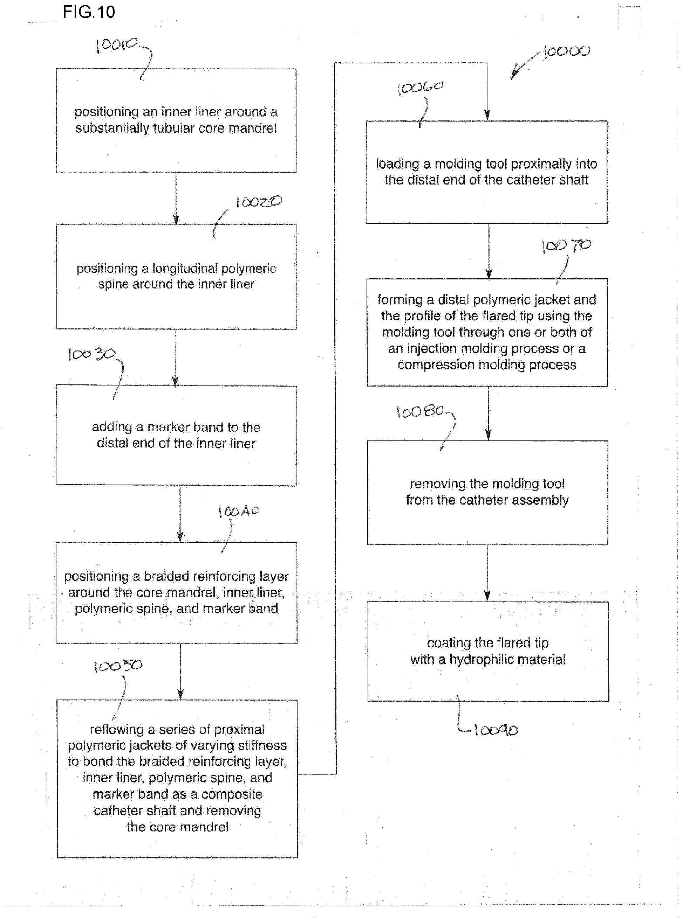

FIG. 10 diagrams method steps for manufacturing an aspiration catheter with the designs disclosed herein. The method steps can be used for any of the example devices or suitable alternatives described herein and known to one of ordinary skill in the art. The method can have some or all of the steps described, and in many cases, steps can be performed in a different order than as disclosed below.

Referring to a method 10000 outlined in FIG. 10, step 10010 can involve positioning an inner liner around a substantially tubular core mandrel. The core mandrel can be used to give structure during manufacturing and define what will be the inner lumen of the catheter. The mandrel can be silver-plated copper (SPC) or other commonly used materials and sized to have an outer diameter such that the resulting catheter has a bore larger than many contemporary aspiration catheters. A strike layer can also be included to better adhere the inner liner to subsequent layers of the catheter shaft.

Step 10020 can include adhering a one or more longitudinal polymeric spines to the inner liner. The spines can be LCP or similar materials with excellent tensile strength to combat the potential for elongation or shortening from loading experienced during a procedure, such as when a fibrin rich clot is dislodged and ingested. The spine or spines can also be of varied cross section, or tapered along their length, to give additional lateral flexibility to distal portions of the catheter shaft. A distal marker band can be added at or near the distal end of the inner liner (step 10030) before or after application of the spine.

The method can further have step 10040 involving positioning a braided reinforcing layer around the core mandrel, inner liner, polymeric spine, and marker band. The braid can have varied PIC count, braid angle, wire thickness, material composition, or other properties to tailor the stiffness profile along the length of the shaft. The components can then be bonded in step 10050 by reflowing a series of proximal polymeric jackets of varying stiffness to mold together the braided reinforcing layer, inner liner, polymeric spine, and marker band as a composite catheter shaft. Once this construct has been set the core mandrel can be removed.

Step 10060 can include loading a molding tool proximally into the distal end of the catheter shaft inside the inner liner. The molding tool can include a profiled die, flared mandrel, or other shape depending on the formation process desired for the distal tip. The molding tool can incorporate features for forming axial ribs, longitudinal and/or radial creases, and distal flanges or lips into the profile of the tip.

Step 10070 can then involve forming a distal polymeric jacket and the profile of the flared tip using the molding tool. As mentioned, the process used can be injection molding, compression molding, reflow, and/or another suitable method. Deponing on the desired shape, or if a reinforcing frame is used for the flared tip, this final distal polymeric jacket can be as soft as approximately 62 Shore A. The use of a soft and very flexible material for the tip can help prevent the device from getting wedged or bound in a vessel when the tip has expanded significantly to accept a large or particularly firm clot.

Depending on the desired shapes, the combination of features in the flared tip can be formed through further steps. In one example, if the tip section is initially reflowed or molded to a reduced thickness, a portion of material at the distal end of the tip can be peeled back over itself and reflowed again to double the wall thickness and add a more rounded end profile. As an alternative, after initial forming of the flared tip a second outer jacket can be positioned over the first and reflowed in place to double the wall thickness.

Other steps and procedures not listed here can be further envisioned by those of skill in the art to form the tip with features and geometry to facilitate expansion and wrap down of the flared tip for the catheter uses described. Upon completion of all the forming processes, step 10080 can then involve removing the flared mandrel from the catheter assembly. A hydrophilic coating can be applied to the formed tip in step 10090.

The invention is not necessarily limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near or a direction towards the physician. Furthermore, the singular forms "a," "an," and "the" include plural referents unless the context clearly dictates otherwise.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

In describing example embodiments, terminology has been resorted to for the sake of clarity. As a result, not all possible combinations have been listed, and such variants are often apparent to those of skill in the art and are intended to be within the scope of the claims which follow. It is intended that each term contemplates its broadest meaning as understood by those skilled in the art and includes all technical equivalents that operate in a similar manner to accomplish a similar purpose without departing from the scope and spirit of the invention. It is also to be understood that the mention of one or more steps of a method does not preclude the presence of additional method steps or intervening method steps between those steps expressly identified. Similarly, some steps of a method can be performed in a different order than those described herein without departing from the scope of the disclosed technology.

## Claims

1. A catheter for administering intravascular treatments, the catheter comprising:
a longitudinal axis;
an elongate body comprising a distal end, a lumen, an inner liner, a reinforcing layer, and one or more outer polymeric jackets; and
a polymeric flared tip connected to the distal end of the elongate body, the flared tip comprising a collapsed delivery configuration, and an expanded deployed configuration, and a plurality of axial ribs.

2. The catheter of claim 1, at least a portion of the flared tip having a wall thickness greater than a wall thickness of at least a portion of the elongate body.

3. The catheter of claim 1, the ratio of an inner diameter of the elongate body to a maximum inner diameter of the flared tip in the expanded deployed configuration being in a range from approximately 0.55 - 0.90.

4. The catheter of claim 1, the reinforcing layer of the elongate body terminating axially at the proximal end of the flared tip.

5. The catheter of claim 1, the flared tip further comprising i) one or more radial creases configured to facilitate folding of the flared tip from the expanded deployed configuration to the collapsed delivery configuration, or ii) one or more longitudinal creases configured to facilitate folding of the flared tip from the expanded deployed configuration to the collapsed delivery configuration.

6. The catheter of claim 1, the flared tip further comprising a maximum inner diameter in the expanded deployed configuration larger than that of an outer sheath through which the catheter is delivered.

7. The catheter of claim 1, each of the one or more polymeric jackets comprising at least one of Esthane with 5% Carbosil, PU 80A, Pebax, Neusoft, Chronosil, Chronoprene, Chronoflex, Tecothane, or silicone.

8. The catheter of claim 1, further comprising a first wall thickness of at least a portion of the flared tip different than a second wall thickness of a different portion of the flared tip.

9. The catheter of claim 1, the flared tip further comprising a maximum inner diameter in a range between approximately 0.088 inches and approximately 0.098 inches in the expanded deployed configuration.

10. The catheter of claim 1, the flared tip tapering at the distal end to a final inner diameter less than a maximum inner diameter of the tip.

11. A catheter for removing a clot from a target blood vessel, the catheter comprising:
a longitudinal axis;
a substantially tubular elongate body comprising a distal end, a reinforcing layer, and one or more polymer jackets;
a metallic support frame connected to the distal end of the elongate body; and
a polymeric flared tip and encapsulating the metallic support frame, the flared tip comprising a collapsed delivery configuration, and an expanded deployed configuration, and a plurality of axial ribs.

12. The catheter of claim 1 or claim 11, the axial ribs comprising i) internal ribs extending radially inward from the inner surface of the flared tip, or ii) external ribs extending radially outward from the outer surface of the flared tip.

13. The catheter of claim 11, the metallic support frame comprising one or more support arms extending distally from the distal end of the elongate body.

14. A method for manufacturing a catheter, the method comprising:
positioning an inner liner around a substantially tubular core mandrel;
positioning a longitudinal polymeric spine around the inner liner;
adding a marker band to the distal end of the inner liner;
positioning a braided reinforcing layer around the core mandrel, inner liner, polymeric spine, and marker band;
reflowing a series of proximal polymeric jackets of varying stiffness to bond the braided reinforcing layer, inner liner, polymeric spine, and marker band as a composite catheter shaft and removing the core mandrel;
loading a molding tool proximally into the distal end of the catheter shaft;
forming a distal polymeric jacket and the profile of the flared tip using the molding tool; and
removing the molding tool from the catheter assembly.

15. The method of claim 14, further comprising the step of forming the flared tip through an injection molding process.

16. The method of claim 14, further comprising the step of forming the flared tip through a compression molding process.
